# EUROPÄISCHE PATENTSCHRIFT

(11) **EP 0 821 952 B1**
(45) Veröffentlichungstag und Bekanntmachung des Hinweises auf die Patenterteilung: **31.03.2004**
(21) Anmeldenummer: 97112938.2
(22) Anmeldetag: 28.07.1997
(51) Int. Cl.: A61K 31/275, A61K 31/42, A61K 31/44, A61K 31/165, A61P 25/00, A61P 37/00, A61P 1/00, A61P 13/12, A61P 9/10

(54) **Verwendung von isoxazol- und Crotonsäureamidderivaten zur Modulation der Apoptose**
Use of isoxazole and crotonic acid amide derivatives for the modulation of apoptosis
L'usage des dérivés d'isoxazole et d'amide d'acide crotonique pour la modulation de l'apoptose

(30) Priorität: 31.07.1996 DE 19630838; 01.10.1996 DE 19640555
(43) Veröffentlichungstag der Anmeldung: 04.02.1998
(73) Patentinhaber: Aventis Pharma Deutschland GmbH, 65929 Frankfurt am Main (DE)
(72) Erfinder: Müllner, Stefan, Dr., 65239 Hochheim (DE); Dax, Claudia, DCh., 64579 Gernsheim (DE)

(56) Entgegenhaltungen:
- EP-A- 0 484 223
- EP-A- 0 529 500
- EP-A- 0 538 783
- EP-A- 0 551 230
- EP-A- 0 665 013
- WO-A-95/19169
- US-A- 4 061 767
- US-A- 5 416 112
- DATABASE WPI Section Ch, Week 199609 Derwent Publications Ltd., London, GB; Class B03, AN 1996-087510 XP002902861 & WO 96 01111 A (WILLIAMS J W), 18. Januar 1996 (1996-01-18)
- DATABASE WPI Section Ch, Week 199429 Derwent Publications Ltd., London, GB; Class B03, AN 1994-236600 XP002902862 & EP 0 607 777 A (HOECHST AG) 27. Juli 1994 (1994-07-27)
- DATABASE WPI Section Ch, Week 199429 Derwent Publications Ltd., London, GB; Class B03, AN 1994-236599 XP002902863 & EP 0 607 776 A (HOECHST AG) 27. Juli 1994 (1994-07-27)
- DATABASE WPI Section Ch, Week 199429 Derwent Publications Ltd., London, GB; Class B03, AN 1994-236598 XP002902864 & EP 0 607 775 A (HOECHST AG) 27. Juli 1994 (1994-07-27)
- DATABASE WPI Section Ch, Week 199429 Derwent Publications Ltd., London, GB; Class B03, AN 1994-236597 XP002902865 & EP 0 607 774 A (HOECHST AG) 27. Juli 1994 (1994-07-27)
- DATABASE WPI Section Ch, Week 199108 Derwent Publications Ltd., London, GB; Class B03, AN 1991-052635 XP002902866 & EP 0 413 329 A (ALCON LAB INC) 20. Februar 1991 (1991-02-20)

## Beschreibung

Bei der Apoptose handelt es sich im Gegensatz zur Nekrose um einen genetisch kontrolliert verlaufenden (programmierten) Zelltod, der essentieller Bestandteil des Lebens mehrzelliger Organismen ist.

Im Gegensatz zu diesem normalen und lebensnotwendigen Apoptoseprozess sind zahlreiche Krankheitsformen oder deren Symptome Ausdruck einer abnormalen, d.h. a) ausufernden oder b) unterdrückten Apoptose [a): Infarkt, Stroke oder Neurodegeneration, b) hypertrophische Erkrankungen]. Heilungsvorgänge von Krankheiten können somit durch Unterdrückung oder Aktivierung der Apoptose möglich sein (z.B. Querschnittslähmung, Immunabwehr usw.). Apoptose verläuft nach Induktion definierter Todessignale, beispielsweise durch Stimulation bestimmter Rezeptoren (z.B. Fas-Rezeptor), über eine sekundär induzierte komplexe Kaskade von ineinandergreifenden biochemischen Ereignissen, an deren Ende die Auflösung der intakten Zelle zu Membran-abgepackten Einheiten steht, die vom Körper ohne oder nur mit geringem Schaden für die umliegenden Zellen (Gegensatz zur Nekrose) entsorgt werden können. Dabei sind in manchen Fällen die Übergänge zwischen Nekrose und Apoptose fließend; so gibt es Fälle, in denen Nekrose zur Apoptose (oder umgekehrt) führt (z.B. Infarkt, Stroke etc.).

Cofilin, ein 19 kDa großes aktinbindendes Protein, spielt als costimulatorischer Faktor in T-Zellen eine entscheidende Rolle bei der Immunreaktion. Cofilin liegt im Cytosol phosphoryliert vor und wird nach Dephosphorylierung in den Zellkern transportiert. Hierbei dient es offenbar als Schleppermolekül für das Protein Aktin, welches keine nukleäre Erkennungssequenz besitzt und als DNAse-I-Inhibitor bekannt ist. Durch diesen Mechanismus kann der Phosphorylierungsgrad des cytosolischen Cofilins einen regulierenden und modulierenden Einfluß auf die Apoptose von Zellen nehmen.

Der Zusammenhang zwischen der Translokation von Aktin-Cofilin-Komplexen im den zellkern und Apoptose wird auch von Y. Samstag et al., The Journal of Immunology, 1996, Seiten 4167-4173, aufgezeigt.

Es wurde nun gefunden, daß Verbindungen der Formel I und II geeignet sind, die Dephosphorylierung von Cofilin zu hemmen und damit haben sie einen modulierenden Einfluß auf die Apoptose.

C.B. Thompson, Science, Band 267, 1995, Seiten 1456-1462, beschreibt, dass durch Modulation der Apoptose eine Reihe von Erkrankungen behandelbar ist.

Die Erfindung betrifft daher die Verwendung von mindestens einer Verbindung der Formel I oder II 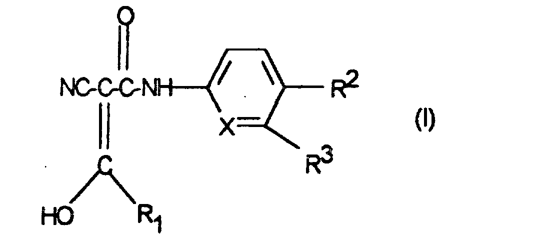 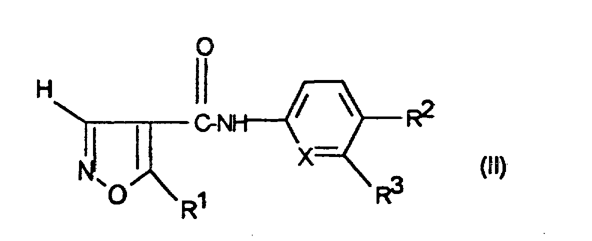 und/oder eine gegebenenfalls stereoisomere Form der Verbindung der Formel I oder II und/oder ein physiologisch verträgliches Salz der Verbindung der Formel I, wobei
R¹ für
a) (C₁-C₄)-Alkyl
b) (C₃-C₅)-Cycloalkyl,
c) (C₂-C₆)-Alkenyl oder
d) (C₂-C₆)-Alkinyl, steht,
R² für
a) -CF₃,
b) -O-CF₃,
c) -S-CF₃,
d) -OH,
e) -NO₂,
f) Halogen,
g) Benzyl,
h) Phenyl,
i) -O-Phenyl,
k) -CN,
l) -O-Phenyl, ein oder mehrfach substituiert mit
   1) (C₁-C₄)-Alkyl,
   2) Halogen,
   3) -O-CF₃ oder
   4) -O-CH₃, steht,
R³ für
a) (C₁-C₄)-Alkyl,
b) Halogen oder
c) ein Wasserstoffatom steht, und
X für
a) eine -CH-Gruppe oder
b) ein Stickstoffatom, steht,
zur Herstellung von Arzneimitteln zur Behandlung von Myomen, Lungensarkoidose, Darmentzündungen, Reperfusionsschäden oder chronischer Niereninsuffizienz.

Bevorzugt ist der Einsatz einer Verbindung der Formel I oder II und/oder eine gegebenenfalls stereosiomere Form der Verbindung der Formel I oder II und/oder ein Salz der Verbindung der Formel I, wobei
R¹ für
a) Methyl,
b) Cyclopropyl oder
c) (C₃-C₅)-Alkinyl steht,
R² für -CF₃ oder -CN steht,
R³ für Wasserstoffatom oder Methyl steht, und
X für eine -CH- Gruppe steht.

Insbesondere bevorzugt ist die Verwendung von N-(4-Trifluormethylphenyl)-2-cyan-3-hydroxy-crotonsäureamid, 2-Cyano-3-cyclopropyl-3-hydroxy-acrylsäure-(4-cyanophenyl)-amid oder N-(4-Trifluormethylphenyl)-2-cyan-3-hydroxy-hept-2-en-6-in-carbonsäureamid.

Die Herstellung der Verbindungen der Formel I und II erfolgt nach bekannten Verfahren wie sie in EP 484 223, EP 529 500, US 4 061 767, EP 538 783 oder EP 551 230 beschrieben werden.

Unter dem Begriff Alkyl, Alkenyl oder Alkinyl werden Reste verstanden, deren Kohlenstoffkette geradkettig oder verzweigt sein kann. Ferner können die Alkenyloder Alkinyl-Reste auch mehrere Doppelbindungen beziehungsweise mehrere Dreifachbindungen enthalten. Cyclische Alkylreste sind beispielsweise 3- bis 5-gliedrige Monocyclen wie Cyclopropyl, Cyclobutyl oder Cyclopentyl.

Die Ausgangsstoffe der chemischen Umsetzungen sind bekannt oder lassen sich nach literaturbekannten Methoden leicht herstellen.

Die erfindungsgemäßen Arzneimittel werden parenteral, oral, rektal oder gegebenenfalls auch topisch appliziert.

Geeignete feste oder flüssige galenische Zubereitungsformen sind beispielsweise Granulate, Pulver, Dragees, Tabletten, (Mikro)Kapseln, Suppositorien, Sirupe, Säfte, Suspensionen, Emulsionen, Tropfen oder injizierbare Lösungen sowie Präparate mit protrahierter Wirkstoff-Freigabe, bei deren Herstellung übliche Hilfsmittel, wie Trägerstoffe, Spreng-, Binde-, Überzugs-, Quellungs-, Gleit- oder Schmiermittel, Geschmacksstoffe, Süßungsmittel oder Lösungsvermittler, Verwendung finden. Als häufig verwendete Hilfsstoffe seien z.B. Titandioxid, Magnesiumcarbonat, Laktose, Mannit und andere Zucker, Talkum, Milcheiweiß, Gelatine, Stärke, Cellulose und ihre Derivate, tierische und pflanzliche Öle, Polyethylenglykole und Lösungsmittel, wie etwa steriles Wasser und ein- oder mehrwertige Alkohole, z.B. Glycerin, genannt.

Aufgrund der pharmakologischen Eigenschaften der Verbindung der Formel I oder II können diese Verbindungen zur gezielten Modulation der Apoptose eingesetzt werden. Daher können Erkrankungen mit ausufemder Apoptose wie Infarkt, Stroke, Transplantationen, Autoimmunerkrankungen, Entzündungen, Neurodegeneration, Myome, Muskelatrophie, Muskeldystrophie, Kachexie, Systemic Inflammation Response Syndrome (SIRS), chronische Lungenentzündung, Adult Respiratory Distress Syndrome (ARDS), zerebrale Malaria, Infarkt, Stroke, Lungensarkosidose, Darmentzündungen, Reperfusionsschäden, Narbenbildung, Verbrennungsschäden, Acquired Immune Deficiency Syndrome (AIDS), Krebs, Erkrankungen mit erhöhten Proteinverlust, chronische Niereninsuffizienz oder hypertrophischen Erkrankungen behandelt werden.

Vorzugsweise wird die Zubereitung in Dosierungseinheiten hergestellt und verabreicht, wobei jede Einheit als aktiver Bestandteil eine bestimmte Dosis von der Verbindung der Formel I und/oder II und/oder physiologisch verträgliche Salze der Verbindung der Formel I enthält. Für die Behandlung eines Patienten (70 kg) sind in frühen Phasen eine intravenöse Infusionsbehandlung von maximal 350 mg pro Tag indiziert. In der späteren Rehabilitationsphase sind zwei bis drei Dosen in einer Menge von 2 mg bis 250 mg, bevorzugt 5 mg bis 150 mg, insbesondere 10 mg bis 50 mg, insbesondere bevorzugt 10 mg bis 20 mg der Verbindung der Formel I und/oder II und/oder der entsprechenden Salze der Verbindung der Formel I indiziert. Die anzuwendende Dosierung ist selbstverständlich abhängig von verschiedenen Faktoren wie dem zu behandelnden Lebewesen (d.h. Mensch oder Tier), Alter, Gewicht, allgemeinen Gesundheitszustand, dem Schweregrad der Symptome, der zu behandelnden Erkrankung, eventuellen Begleiterkrankungen (falls vorhanden), der Art der begleitenden Behandlung mit anderen Arzneimitteln, oder Häufigkeit der Behandlung. Die Dosierungen werden im allgemeinen mehrfach pro Tag und vorzugsweise einmal bis dreimal pro Tag verabreicht. Die verwendeten Mengen an Verbindung der Formel I orientieren sich hierbei an der empfohlenen Tagesdosis der jeweiligen Verbindung der Formel I oder II und der Löslichkeit der Verbindung der Formel I oder II.

Ferner können die Verbindungen der Formel I oder II auch zusammen mit anderen geeigneten Wirkstoffen, beispielsweise Antiuricopathika, Analgetika, steroidalen oder nichtsteroidalen Antiphlogistika, Thrombocytenaggregationshemmern oder immunsuppressiven Verbindungen wie Cylosporin A, FK 506 oder Rapamycin eingesetzt werden.

### Beispiel 1

### Pharmakologische Prüfung

### 1.1 Zellkultur

Die murine Makrophagenzellinie RAW 264.7 wurde von ATCC (Rockville, MD) bezogen und in DMEM (Sigma, St. Louis, MO) mit 4,5 g Glucose/l, 110 mg Natriumpyruvat/l, 10 % Hitze inaktiviertem FCS (Gibco, Grand Island, NY) und Penicillin/Streptomycin (50 U/50 mg/ml) kultiviert. Die Makrophagen wurden alle 2 - 3 Tage passagiert und einen Tag vor Beginn des Experiments zu 2*10⁶ Zellen in Gewebekulturflaschen (75 cm², Falcon, Becton Dickinson GmbH, Heidelberg, Deutschland) ausgebracht. Die Zellen wurden mit frischem Medium versorgt und die Präparate in den entsprechenden Konzentrationen zugesetzt. N-(4-Trifluormethylphenyl)-2-cyan-3-hydroxy-crotonsäureamid-Natriumsalz (Verbindung 1) wurde 12 mM in Zellmedium gelöst. Davon wurden je 100 µl (60 µM Endkonzentration), 33 µl (20 µM Endkonzentration) und 16,7 µl (10 µM Endkonzentration) zu 20 ml Medium pipettiert. Die Stimulation mit Lipopolysaccharid (LPS; E. coli, Serotype 0127:B 8; Sigma, St. Louis, MO) in einer Konzentration von 10 ng/ml wurde 1 Stunde nach der Vorinkubation mit dem Präparat durchgeführt.

Aliquotes einer Stammlösung von Lipopolysacchariden (LPS ; 1 mg/ml in 10 % DMSO) wurden mit Medium auf eine Konzentration von 1 µg/ml verdünnt und bei -20°C gelagert. Die Zellen wurden für 24 Stunden (h) bei 37°C in 10 % CO₂ inkubiert.

### 1.2 Probenvorbereitung

Alle verwendeten Chemikalien waren analytisch rein oder in Elektrophoresequalität und wurden von Millipore Co. (Bedford, MA) oder Sigma (St. Louis, MO) bezogen, wenn nicht gesondert auf andere Bezugsquellen hingewiesen wird.

Die 2-D-Elektrophorese (2-DE) wurde mit dem Investigator System® (Millipore) durchgeführt, und die Proben wurden nach der Vorschrift des Herstellers mit kleinen Veränderungen aufgearbeitet. Die adhärenten murinen Makrophagen wurden auf Eis stehend dreimal je 60 Sekunden mit 10 ml eiskaltem PBS gewaschen. Anschließend wurden die Zellen in 1 ml kochendem Lysispuffer, bestehend aus 0,3 g SDS, 3,088 g DTT, 0,444 g TrisHCl und 0,266 g Tris Base, in 100 ml lysiert. Das Zellysat wurde abgeschabt und in einem 2 ml Probengefäß für 10 Minuten (min) in kochendem Wasser erhitzt.
Polynucleotide wurden durch Zusatz von Benzonase® (Merck, Darmstadt, Deutschland) in 30 min bei 37 °C gespalten. An dieser Stelle der Probenvorbereitung wurde ein Aliquot entnommen, und der Proteingehalt nach der Methode von Popov bestimmt.

Für die 2-DE wurden die Proteine der Probe durch tropfenweise Zugabe zu eiskaltem Aceton (80 % v/v) ausgefällt. Die Probe wurde für 20 min auf Eis gekühlt und anschließend bei 240 g 10 min zentrifugiert. Das angetrocknete Pellet wurde in einem Teil Lysispuffer und vier Teilen eines Probenpuffers zu einem Proteingehalt von 5 mg/ml aufgenommen. Der Probenpuffer besteht aus 59,7g Harnstoff, 4,0 ml NP-40, 1,54 g DTT, 5,5 ml Trägerampholyten (pH 3-10, 2-DE optimiert) in 100 ml. Ungelöstes Material wurde vor der Elektrophorese durch Zentrifugation der Proben bei 16000 x g abgetrennt.

### 1.3 2-DE Gel-Elektrophorese

Die hochauflösende Zwei-Dimensionale Gel-Elektrophorese wurde nach der Methode von O'Farrell mit Modifikationen, wie sie von Garrels beschrieben wurden, durchgeführt. Dazu wurde das Millipore Investigator® 2-D Elektrophorese System (Millipore Co., Bedford, MA) eingesetzt.

Die isoelektrische Fokussierung wurde in Glaskapillaren (1 mm im Durchmesser) mit einem 0,08 mm dicken Faden, der ein Dehnen und Reißen des Stäbchens verhindert, durchgeführt. Das IEF-Gel besteht aus einer 4,1 % T, 2,4 % C Polyacrylamidmatrix, die aus einer 30,8 % T, 2,6 % C Stammlösung hergestellt wurde, 9,5 M Harnstoff, 2,0 % (v/v) NP-40, 10 mM Chaps und 2 % (v/v) Trägerampholyten (pH 3-10, 2-DE optimiert).
Als Anodenpuffer wurde 0,01 M H₃PO₄, als Kathodenpuffer 0,1 M NaOH benutzt Vor der Vorfokussierung zur Ausbildung des pH-Gradienten wurden 15 µl eines Probenüberschichtungspuffers, bestehend aus 0,5 M Harnstoff, 0,2 % (v/v) NP-40, 0,1 % (v/v) Trägerampholyten und 50 mM DTT, appliziert. Das Spannungsmaximum von 1500 Volt wurde innerhalb von 90 Minuten bei einem maximalen Strom von 110 µA/Gel erreicht. Nach der Vorfokussierung wurden 20 µl der Probe (100 µg Protein) und weitere 15 µl Überschichtungspuffer aufgetragen.

Die isoelektrische Fokussierung der Proteine erfolgte innerhalb von 18000 Vh. Nach Beendigung der Elektrophorese wurden die Stäbchen auf Eis gekühlt und in einem Puffer, bestehend aus 0,3 M Tris Base, 0,075 M Tris HCl, 6 % SDS, 50 mM DTT und 0,01 % Bromphenolblau, äquilibriert. Die Stäbchengele wurden direkt auf die Oberfläche des Vertikalgels der zweiten Dimension überführt oder bis zum Gebrauch bei - 20 °C gelagert. Die zweite Dimension wurde in einem SDS-Gradientengel (10 - 17 %) ohne Sammelgel durchgeführt. Der Gradient wurde durch Mischen zweier Gellösungen hergestellt.
A: 100 ml Acrylamid (30,5 % T, 1,64 % C), 73 ml Tris (1,5 M, pH 8,8), 123 ml H₂O, 3 ml SDS (10 %), 150 µl TEMED und 750 µl Ammoniumperoxodisulfat (10 %).
B: 170 ml Acrylamid, 73 ml Tris, 66,78 g Glycerin, 3 ml SDS, 150 µl TEMED, 750 µl Ammoniumperoxodisulfat.

Die Elektrophorese wurde über Nacht bei konstanter Temperatur in einem Laufpuffer, bestehend aus 25 mM Tris-Base, 192 mM Glyzin und 0,1 % SDS, durchgeführt bis die Bromphenolblaufront etwa 1 cm vom Ende des Gels entfernt war. Nach Beendigung der Elektrophorese wurden die Proteine im Gel nach Heukeshoven und Dernick mit Silberreagenz angefärbt.

Die Analyse der 2-D-Gele und die Herstellung synthetischer Bilder wurde mit dem Biolmage System (Biolmage Systems Co.) durchgeführt. Das erhaltene Proteinmuster wurde von einer Kodak Megaplus Camera Model 1,4 gescannt und die Daten wurden von einer HAM-Station prozessiert.

### 1.4 Ergebnisse

Die Ergebnisse der unstimulierten Kontrolle wurden gleich 100 % gesetzt. Die Zugabe von LPS (10 ng/ml) führte zu einer 50 % Dephosphorylierung von Cofilin. Die gleichzeitige Applikation von LPS (10 ng/ml) und Verbindung 1 (60 µM) führte dagegen zu keiner Dephosphorylierung von Cofilin. Daher ergab sich in Anwesenheit von Verbindung 1 eine 100 %ige Hemmung der Dephosphorylierung von Cofilin in den Makrophagen im Vergleich mit der Hemmung, die bei den nur mit LPS behandelten Makrophagen erzielt wurde.

Die Zugabe von LPS (10 ng/ml) und 20 µM Verbindung 1 oder 10 µM Verbindung 1 ergab dieselbe Dephosphorylierung von Cofilin wie ohne Zugabe von Verbindung 1. Daher führen 20 µM oder 10 µM der Verbindung 1 nicht mehr zu einer Hemmung der Dephosphorylierung von Cofilin.

## Patentansprüche

1. Verwendung von mindestens einer Verbindung der Formel I und/oder II 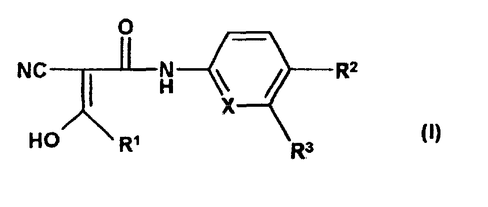 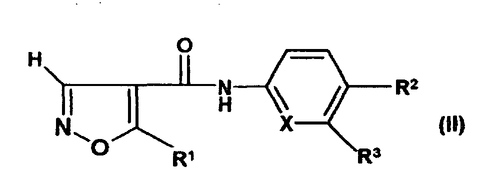 und/oder einer gegebenenfalls stereoisomeren Form der Verbindung der Formel I oder II und/oder einem physiologisch verträglichen Salz der Verbindung der Formel I, wobei
R¹ für
a) (C₁-C₄)-Alkyl
b) (C₃-C₅)-Cycloalkyl,
c) (C₂-C₆)-Alkenyl oder
d) (C₂-C₆)-Alkinyl, steht,
R² für
a) -CF₃,
b) -O-CF₃,
c) -S-CF₃,
d) -OH,
e) -NO₂,
f) Halogen,
g) Benzyl,
h) Phenyl,
i) -O-Phenyl,
k) -CN,
l) -O-Phenyl, ein oder mehrfach substituiert mit
1) (C₁-C₄)-Alkyl,
2) Halogen,
3) O-CF₃ oder
4) -O-CH₃, steht,
R³ für
a) (C₁-C₄)-Alkyl,
b) Halogen oder
c) ein Wasserstoffatom steht, und
X für
a) eine -CH-Gruppe oder
b) ein Stickstoffatom, steht
zur Herstellung eines Arzneimittels zur Behandlung von Myomen, Lungensarkoidose, Darmentzündungen, Reperfusionsschäden oder chronischer Niereninsuffizienz.

2. Verwendung zur Herstellung eines Arzneimittels gemäß Anspruch 1, **dadurch gekennzeichnet, dass** es eine Verbindung der Formel I und/oder II und/oder eine gegebenenfalls stereoisomere Form der Verbindung der Formel I oder II und/oder ein Salz der Verbindung der Formel I, wobei
R¹ für
a) Methyl,
b) Cyclopropyl oder
c) (C₃-C₅)-Alkinyl steht,
R² für CF₃ oder CN steht,
R³ für ein Wasserstoffatom oder Methyl steht, und
X für eine -CH-Gruppe steht,
enthält.

3. Verwendung zur Herstellung eines Arzneimittels gemäß Anspruch 1 oder 2, **dadurch gekennzeichnet, dass** es N-(4-Trifluormethylphenyl)-2-cyan-3-hydroxy-crotonsäureamid, 2-Cyano-3-cyclopropyl-3-hydroxy-acrylsäure-(4-cyanophenyl)-amid oder N-(4-Trifluormethylphenyl)-2-cyan-3-hydroxy-hept-2-en-6-in-carbonsäureamid enthält.

4. Verwendung zur Herstellung eines Arzneimittels gemäß einem oder mehreren der Ansprüche 1 bis 3, **dadurch gekennzeichnet, dass** die Verbindung der Formel I und/oder II in einer Dosis von 2 mg bis 250 mg, insbesondere von 10 mg bis 50 mg, verabreichbar ist.

5. Verwendung zur Herstellung eines Arzneimittels gemäß einem oder mehreren der Ansprüche 1 bis 4, **dadurch gekennzeichnet, dass** es zusätzlich Antiuricopathika, Analgetika, steroidale oder nichtsteroidale Antiphlogistika, Thrombocytenaggregationshemmer oder immunsuppressive Verbindungen enthält.

6. Verwendung zur Herstellung eines Arzneimittels gemäß Anspruch 5, **dadurch gekennzeichnet, dass** es zusätzlich Cyclosporin A, FK 506 oder Rapamycin enthält.

## Claims

1. The use of at least one compound of the formula I and/or II 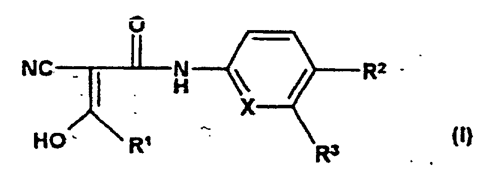 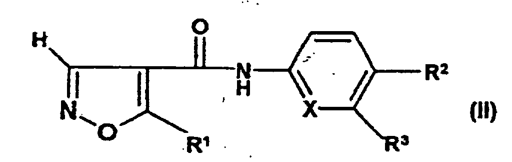 and/or an optionally stereoisomeric form of the compound of the formula I or II and/or a physiologically tolerable salt of the compound of the formula I, where
R¹ is
a) (C₁-C₄)-alkyl
b) (C₃-C₅)-cycloalkyl,
c) (C₂-C₆)-alkenyl or
d) (C₂-C₆)-alkynyl,
R² is
a) -CF₃,
b) -O-CF₃,
c) -S-CF₃,
d) -OH,
e) -NO₂,
f) halogen,
g) benzyl,
h) phenyl,
i) -O-phenyl,
k) -CN or
l) -O-phenyl, mono- of polysubstituted by
1) (C₁-C₄)-alkyl,
2) halogen,
3) -O-CF₃ or
4) -O-CH₃,
R³ is
a) (C₁-C₄)-alkyl,
b) halogen, or
c) a hydrogen atom, and
X is
a) a -CH group or
b) a nitrogen atom,
for the production of a pharmaceutical for the treatment of myomata, pulmonary sarcosidosis, enteritis, reperfusion damage or chronic renal insufficiency.

2. The use for the production of a pharmaceutical as claimed in claim 1, **characterized in that** said pharmaceutical comprises a compound of the formula I and/or II and/or an optionally stereoisomeric form of the compound of the formula I or II and/or a salt of the compound of the formula I, where
R¹ is
a) methyl,
b) cyclopropyl or
c) (C₃-C₅)-alkynyl,
R² is CF₃ or CN,
R³ is a hydrogen atom or methyl, and
X is a -CH- group.

3. The use for the production of a pharmaceutical as claimed in claim 1 or 2, **characterized in that** said pharmaceutical comprises N-(4-trifluoromethylphenyl)-2-cyano-3-hydroxycrotonamide, 2-cyano-3-cyclopropyl-3-hydroxyacrylic acid (4-cyanophenyl)amide or N-(4-trifluoromethylphenyl)-2-cyano-3-hydroxyhept-2-en-6-yne-carboxamide.

4. The use for the production of a pharmaceutical as claimed in one or more of claims 1 to 3, **characterized in that** the compound of the formula I and/or II is administrable in a dose of 2 mg to 250 mg, in particular of 10 mg to 50 mg.

5. The use for the production of a pharmaceutical as claimed in one or more of claims 1 to 4, **characterized in that** said pharmaceutical further comprises antiuricopathics, analgesics, steroidal or nonsteroidal antiinflammatories, platelet aggregation inhibitors or immunosuppressant compounds.

6. The use for the production of a pharmaceutical as claimed in claim 5, **characterized in that** said pharmaceutical further comprises cyclosporin A, FK 506 or rapamycin.

## Revendications

1. Utilisation, pour préparer un médicament destiné au traitement des myomes, de la sarcoidose pulmonaire, des inflammations gastriques, des lésions de reperfusion ou de l'insuffisance rénale chronique, d'au moins un composé de formule I et/ou II 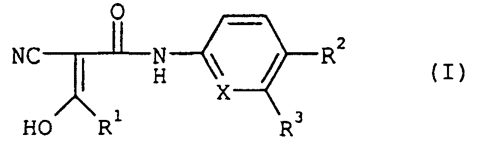 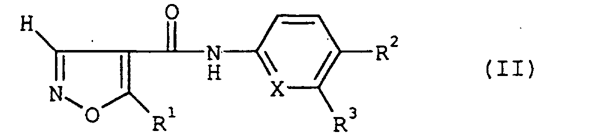 et/ou d'une forme éventuellement stéréoisomère du composé de formule I ou II, et/ou d'un sel acceptable d'un point de vue physiologique du composé de formule I, où
R¹ représente un groupe
a) alkyle en C₁-C₄,
b) cycloalkyle en C₃-C₅,
c) alcényle en C₂-C₆, ou
d) alcynyle en C₂-C₆,
R² représente un groupe
a) -CF₃,
b) -O-CF₃,
c) -S-CF₃,
d) -OH,
e) -NO₂,
f) halogéno,
g) benzyle,
h) phényle,
i) -O-phényle,
k) -CN,
l) -O-phényle, une ou plusieurs fois substitué par des substituants
1) alkyle en C₁-C₄,
2) halogéno,
3) O-CF₃ ou
4) -O-CH₃,
R³ représente un groupe
a) alkyle en C₁-C₄,
b) halogéno, ou
c) un atome d'hydrogène, et
X représente
a) un groupe -CH, ou
b) un atome d'azote.

2. Utilisation pour préparer un médicament selon la revendication 1, **caractérisé en ce qu'**il contient un composé de formule I et/ou II et/ou une forme éventuellement stéréoisomère du composé de formule I ou II et/ou un sel du composé de formule I, où
R¹ représente un groupe
a) méthyle,
b) cyclopropyle, ou
c) alcynyle en C₃-C₅,
R² représente CF₃ ou CN,
R³ représente un atome d'hydrogène ou le groupe méthyle, et
X représente un groupe -CH.

3. Utilisation pour préparer un médicament selon la revendication 1 ou 2, **caractérisé en ce qu'**il contient du N-(4-trifluorométhylphényl)-2-cyano-3-hydroxycrotonamide, du (4-cyanophényl)-amide de l'acide 2-cyano-3-cyclopropyl-3-hydroxy-acrylique, ou du N-(4-trifluorométhylphényl)-2-cyano-3-hydroxy-hept-2-ène-6-yne-carboxamide.

4. Utilisation pour préparer un médicament selon l'une ou plusieurs des revendications 1 à 3, **caractérisé en ce que** le composé de formule I et/ou II peut être administré à une dose de 2 mg à 250 mg, en particulier de 10 mg à 50 mg.

5. Utilisation pour préparer un médicament selon l'une ou plusieurs des revendications 1 à 4, **caractérisé en ce qu'**il contient en outre des antigoutteux, des analgésiques, des antiphlogistiques stéroidiens ou non stéroidiens, des inhibiteurs de l'agrégation thrombocytaire ou des composés immunosuppresseurs.

6. Utilisation pour préparer un médicament selon la revendication 5, **caractérisé en ce qu'**il contient en outre de la cyclosporine A, du FK 506 ou de la rapamycine.
